# EUROPEAN PATENT APPLICATION

(11) **EP 4 029 638 A1**
(43) Date of publication of application: **20.07.2022**
(21) Application number: 20863810.6
(22) Date of filing: 18.08.2020
(51) Int. Cl.: B23C 3/16, B23C 3/04, B23P 15/00

(54) **DIMPLE MACHINING METHOD**

(30) Priority: 13.09.2019 JP 2019167563
(71) Applicant: Kanefusa Kabushiki Kaisha, Ohguchi-cho, Niwa-gun Aichi 480-0192 (JP)
(72) Inventor: SATO Toshiki, Niwa-gun, Aichi 480-0192 (JP); KANDA Yasuyuki, Niwa-gun, Aichi 480-0192 (JP); NIIMI Tatsuya, Niwa-gun, Aichi 480-0192 (JP)
(74) Representative: Maikowski & Ninnemann Patentanwälte Partnerschaft mbB
(86) International application number: PCT/JP2020/031089
(87) International publication number: WO 2021/049260

(57) **Abstract**

In a dimple processing method for forming dimples on a curved surface (41) of a workpiece (40), a cutting tool 1 having a cutting edge (5a) on a rod-shaped body (2) is rotated about an axis (2a). The workpiece (40) is rotated about a first axis (20a). A cutting tool (1) or a workpiece (40) moves on a virtual plane orthogonal to the Y-axis and through which the first axis (20a) passes or on a plane parallel to the virtual plane such that the tip end (3) of the cutting tool (1) follows the curved surface (41) of the workpiece (40). The cutting edge (5a) forms dimples on the curved surface (41) each time the cutting tool 1 rotates.

## Description

### Technical Field

One embodiment of the present disclosure relates to a dimple processing method for forming dimples on a curved surface of a workpiece.

### Background Art

Dimples, which are a plurality of fine recesses, may be formed on a surface of workpieces made of, for example, aluminum, copper alloys, castings made thereof, steel materials, or resins. For example, dimples may be formed on a metal workpiece by scraping. A satin pattern may be formed on a surface of a workpiece using a plurality of dimples. This is done because the frictional resistance generated between a counter material in contact with the workpiece and the workpiece can be reduced by forming dimples on the workpiece. The principle is that, for example, when a workpiece and a counter material come into contact with each other, abrasion debris is generated. The abrasion debris may be captured between the workpiece and the counter material resulting in an increase in the frictional resistance. By allowing this abrasion debris to be accumulated in the dimples, the frictional resistance due to the abrasion debris may be suppressed. Alternatively, water or oil may be injected between the workpiece and the counter material so as to fill the dimples with water or oil. When the counter material passes near the dimples, water or oil is squeezed out of the dimples at high pressure and in between the counter material and the workpiece (squeeze effect). This pressure may hinder the counter material from coming into contact with the workpiece, thereby reducing the frictional resistance between the counter material and the workpiece.

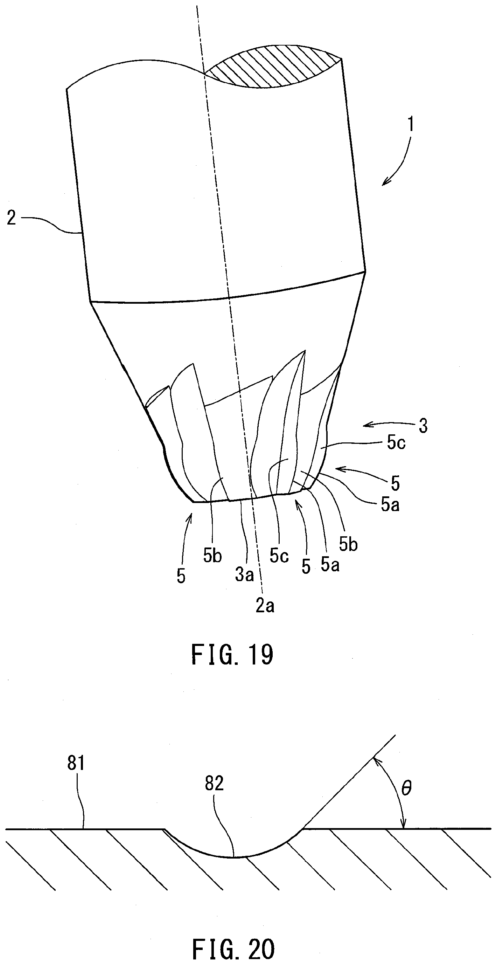

Therefore, dimples may be formed on inner walls of tubular members, such as engine cylinders and turbochargers, and joint surfaces of artificial joints. U.S. Pat. No. 9,872,772 discloses an artificial joint in which dimples are formed on a spherical joint surface. Conventional dimples were formed by, for example, laser machining, shot blasting, etching, press molding, or the like. In these processing methods, it is difficult to reduce an approach angle θ of the dimples 82 formed on the surface 81, as shown in FIG 20. FIG 20 schematically shows one of the present embodiments, instead of a conventional structure. In addition, the surface of the workpiece may be altered or deformed due to heat generated during processing, residual stress, or the like. If the approach angle of the dimple is increased, the entry of water or oil into the dimples is hindered, such that the affinity (hydrophilicity, lipophilicity) may decrease (pinning effect). Therefore, it may be difficult to reduce the frictional resistance of the dimpled workpiece. In the case of shot blasting or press molding, it is difficult to reproduce the shape of the dimples with high accuracy.

JPH10-052998A discloses a method of decorating the surface of a workpiece by using a cutting tool, such as a milling cutter or an end mill. In this method, the cutting edge of the cutting tool is brought barely in contact with the surface of the workpiece while rotating the cutting tool. As a result, a plurality of dimples having, for example, a circular shape or an elliptical shape can be formed on the surface of the workpiece in a polka dot pattern. By machining the workpiece with a cutting tool, the dimple approach angle can be set small. This may also reduce burrs, heat, residual stress, etc. generated during processing. Accordingly, the surface condition of the dimpled workpiece can be easily finished with a high quality without the need to perform a finishing processing, such as a lapping process. When machining dimples on a flat surface, a cylindrical surface, or the like of a workpiece, the dimples can be successively machined along a feeding direction relative to the cutting tool of the workpiece. Therefore, a plurality of dimples can be processed into the workpiece in a relatively short period of time while maintaining a good surface condition of the workpiece.

### Summary of Invention

### Technical Problem

It is also possible to allow an end mill to move forward and backward in the axial direction to process dimples on a curved surface of a workpiece. However, this processing method requires a lot of time to form a plurality of dimples. Therefore, there has been a need for providing a processing method for forming a plurality of dimples having a simple and suitable shape on a curved surface for a long time.

### Solution to Problem

One aspect of the present disclosure relates to a dimple processing method for forming dimples on a curved surface of a workpiece. A cutting tool having a cutting edge on a rod-shaped body is rotated around the axis of the rod-shaped body. The workpiece is rotated around a first axis of the workpiece. The cutting tool or the workpiece is moved on a virtual plane through which the first axis passes or on a plane parallel to the virtual plane such that the tip end of the cutting tool follows the curved surface of the workpiece. The cutting edge forms dimples on the curved surface each time the cutting tool rotates.

Therefore, the cutting tool or workpiece is configured to move on a flat surface, that is, in two dimensions. The cutting tool and the workpiece are rotated around their respective axes. As a result, a plurality of dimples can be formed on a curved surface in a short period of time. Moreover, the depth of the dimples can be made substantially constant and the approach angle can be reduced. By making the depth substantially constant, it is easy to reproduce a plurality of dimples with the same shape and the same size. By reducing the approach angle, burrs, heat, residual stress, etc. generated during processing can be reduced.

Another aspect of the present disclosure relates to a dimple processing method for forming dimples on a curved surface of a workpiece. A cutting tool having a cutting edge on the rod-shaped body is rotated around the axis of the rod-shaped body. The cutting tool or workpiece is configured to move in three directions orthogonal to each other, such that the tip end of the cutting tool follows the curved surface of the workpiece without rotating the workpiece. The cutting edge forms dimples on the curved surface each time the cutting tool rotates.

Therefore, the cutting tool or workpiece is moved in three dimensions. Only the cutting tool is rotated around the axis. The workpiece is not rotated. This allows a plurality of dimples to be formed on the curved surface in a short period of time. Moreover, the depth of the dimples can be made substantially constant and the approach angle can be reduced.

According to another aspect of the present disclosure, the cutting edge extends continuously along the longitudinal direction of the rod-shaped body. While maintaining the angle of the rod-shaped body of the cutting tool with respect to the workpiece, the rod-shaped body moves relative to the workpiece. This causes a change in the contact point of the cutting edge with respect to the curved surface. Therefore, it is possible to avoid concentrating the cutting force on only a single part of the cutting edge by changing the contact point. As a result, the life of the cutting edge can be extended.

According to another aspect of the present disclosure, the cutting tool is inclined with respect to the workpiece so that the angle of the rod-shaped body is constant with respect to the surface of the cutting tool in contact with the curved surface of the workpiece. As a result, the contact point of the cutting edge with respect to the curved surface is kept constant. Therefore, the approach angle and cutting depth of the cutting edge with respect to the curved surface can be made constant. This allows a plurality of dimples having the same shape and the same size to be repeatedly formed.

Another aspect of the present disclosure relates to a dimple processing method for forming dimples on a curved surface of a workpiece. The curved surface has a partial spherical shape, and the workpiece is rotated around a first axis extending through the center of the spherical surface. The workpiece is rotated around the second axis orthogonal to the first axis. A cutting tool having a cutting edge on the rod-shaped body is rotated around the axis of the rod-shaped body. The tip end of the cutting tool moves along a spherical portion of the workpiece. The cutting edge forms dimples on the curved surface each time the cutting tool rotates.

Therefore, the workpiece and cutting tool are rotated around three axes in total. As a result, a plurality of dimples can be formed on the spherical portion of the curved surface in a short period of time. The spherical partial shape may be either convex or concave. Moreover, the depth of the dimples can be made substantially constant and the approach angle can be reduced.

Another aspect of the present disclosure relates to a dimple processing method for forming dimples on a curved surface of a workpiece. Changing the moving speed and the amount of movement of the cutting tool relative to the curved surface causes the intervals between the plurality of dimples and the cutting depth to have a predetermined size in accordance with the relative movement of the cutting tool with respect to the curved surface. Therefore, it is possible to change the interval and depth of the plurality of dimples per each part of the curved surface.

### Brief Description of Drawings

FIG. 1 is a perspective view of a processing machine and a workpiece holding device.
FIG. 2 is a perspective view of a part of the processing machine and a part of the workpiece holding device when a table is tilted.
FIG. 3 is a perspective view of a cutting tool.
FIG. 4 is a bottom view of the cutting tool.
FIG. 5 is a side view of a part of a lateral side of the cutting tool and a part of a cross section of a convex curved surface of the workpiece.
FIG. 6 is a side view of a part of a lateral side of the cutting tool and a part of a cross section of a concave curved surface of the workpiece.
FIG. 7 is a block diagram of a controller for the processing machine and the workpiece holding device.
FIG. 8 is a side view of a part of the processing machine and a part of the workpiece holding device when forming dimples on the curved surface of the workpiece at a constant tilt angle of an axis of the cutting tool.
FIG. 9 is a perspective view of the curved surface with dimples formed and arranged in a spiral manner.
FIG. 10 is an enlarged schematic view of a part X in FIG. 9.
FIG. 11 is a side view of a part of the processing machine and a part of the workpiece holding device when forming dimples on the curved surface of the workpiece at a constant lead angle of the axis of the cutting tool.
FIG. 12 is a side view of a part of the processing machine and a part of the workpiece holding device when forming dimples on the curved surface of the workpiece with the cutting tool shifted and the axis inclined.
FIG. 13 is a side view of a part of the processing machine and a part of the workpiece holding device when forming dimples on the curved surface of the workpiece by shifting the cutting tool while the direction of the axis remains constant.
FIG. 14 is a side view of a part of the processing machine and a part of the workpiece holding device when forming dimples on the curved surface of the workpiece by turning the table.
FIG. 15 is a side view of a part of the processing machine and a part of the workpiece holding device when forming dimples on the curved surface of the workpiece by shifting the cutting tool.
FIG. 16 is a block diagram of a controller for the processing machine and the workpiece holding device.
FIG. 17 is an enlarged schematic view of a part Y in FIG. 21.
FIG. 18 is a perspective view of the curved surface with dimples formed and arranged in a spiral manner centered on the locations where the dimples are shifted from the apex of the curved surface.
FIG. 19 is a side view of a tip end of the cutting tool having a plurality of cutting edges.
FIG. 20 is a schematic cross-sectional view illustrating an approach angle of a dimple formed on a surface.
FIG. 21 is a perspective view of the curved surface with dimples arranged in a lattice pattern.

### Description of Embodiments

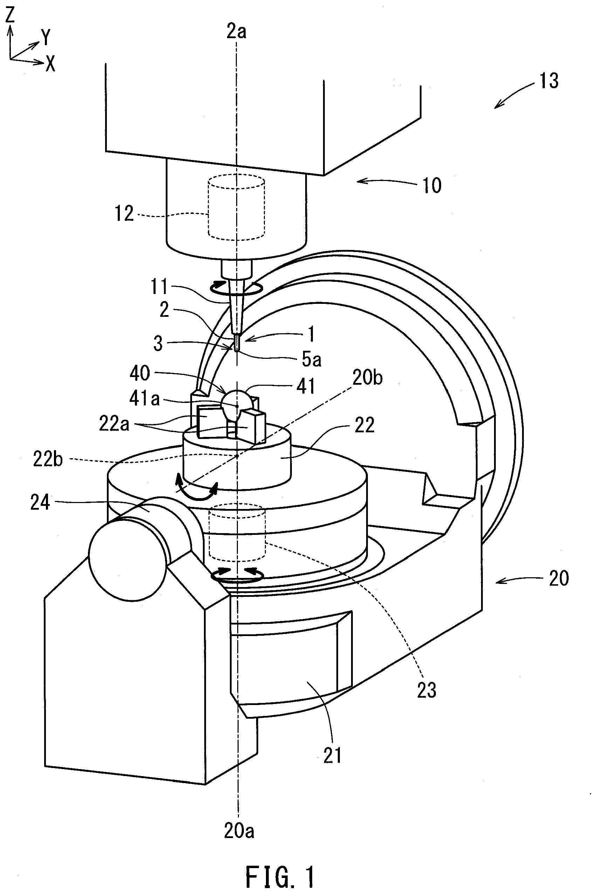

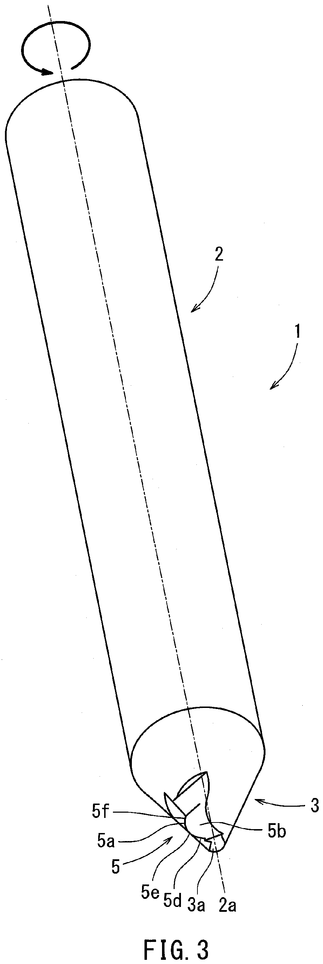

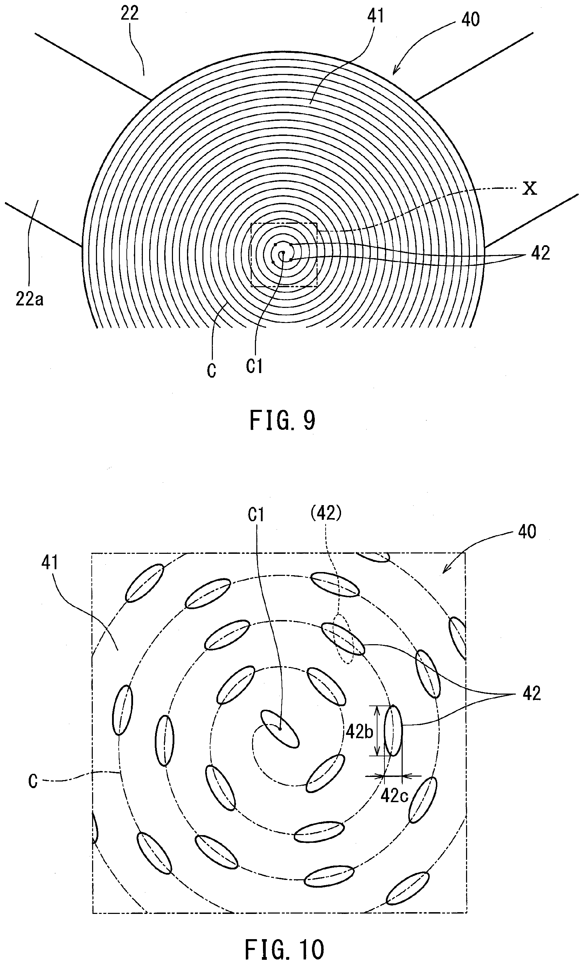

Hereinafter, one exemplary embodiment of the present disclosure will be described with reference to FIGS. 1 to 11. A cutting tool 1 shown in FIG. 3 is a cutting tool for forming a plurality of separated dimples (fine recesses) 42 on a workpiece 40 as shown in FIG. 10. The dimples 42 are formed by a dimple processing machine 13 that utilizes the cutting tool 1, as shown in FIG. 1. The dimple processing machine 13 includes a processing apparatus 10 on which the cutting tool 1 is attached and a workpiece holding device 20 on which the workpiece 40 is placed. For example, the processing apparatus 10 is arranged on the upper side, and the workpiece holding device 20 is arranged below the processing apparatus 10.

As shown in FIG. 3, the cutting tool 1 includes a rod-shaped body 2 and a tip end 3 provided at the leading end of the rod-shaped body 2. The rod-shaped body 2 may have, for example, a round rod shape or a columnar shape. The axis 2a of the rod-shaped body 2 is located at the center of the cross section of the rod-shaped body 2 and extends in a longitudinal direction. The axis 2a is the center of rotation of the cutting tool 1. The tip end 3 is formed in a conical shape centered on the axis 2a. The diameter of the tip end 3 decreases toward the leading end of the rod-shaped body 2. A flat top 3a passing across the axis 2a is formed at the end of the tip end 3.

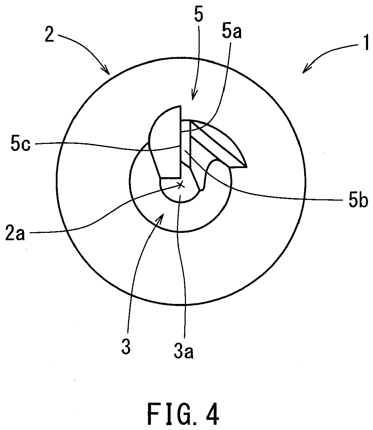

As shown in FIGS. 3 and 4, the tip end 3 has one cutting edge portion 5. The cutting edge portion 5 projects in the radial direction of the rod-shaped body 2 from the peripheral edge of the tip end 3. The cutting edge 5a of the cutting edge portion 5 has an arc shape protruding in the radial direction and from the rod-shaped body 2. A cutting edge end portion 5d of the cutting edge 5a projects from the top portion 3a in the radial direction of the rod-shaped body 2. The cutting edge 5a has a radial tip end portion 5e at the most protruding location in the radial direction of the rod-shaped body 2. The cutting edge 5a has a cutting edge base portion 5f that is in contact with the tip end 3 closer to the base side of the rod-shaped body 2 than to the radial tip end portion 5e.

As shown in FIGS. 3 and 4, the cutting edge portion 5 has a rake face 5c closer to the rotation direction side (left side in FIG. 4) of the cutting tool 1 than the cutting edge 5a. The rake angle of the rake face may be, for example, -20 to 10°. Further, a flank 5b is provided on the opposite side of the rake face 5c (to the right of the cutting edge 5a in FIG. 4). The flank 5b is generally oriented outward in the radial direction of the rod-shaped body 2. The cutting edge 5a of the ridge line defined by the rake face 5c and the flank 5b has substantially an arc shape.

The cutting edge portion 5 shown in FIG. 3 may be formed of the same material as the rod-shaped body 2 of the cutting tool 1, or may be formed of a different material. For example, the cutting edge portion 5 and the rod-shaped body 2 may be formed of tool steel, high-speed steel (high-speed tool steel), or cemented carbide. Alternatively, the rod-shaped body 2 may be formed of carbon steel, stainless steel, tool steel, high-speed steel, or cemented carbide, and the cutting edge portion 5 may be formed of a monocrystalline diamond (MCD), a polycrystalline diamond (PCD), cubic boron nitride (CBN) or ceramics, and the cutting edge portion 5 is joined to the rod-shaped body 2. Alternatively, the cutting edge portion 5 may be formed of the same or different material as the rod-shaped body 2, and the area corresponding to the cutting edge portion 5 is subjected to surface treatment such as coating. The surface treatment may be performed by, for example, a chemical vapor deposition method (CVD) or a physical vapor deposition method (PVD), and the coating layer such as Ti-based materials such as TiAIN, TiAlCrN and TiAlCrSiN, a CVD diamond and diamond-like carbon (DLC) may be used as the cutting edge portion 5.

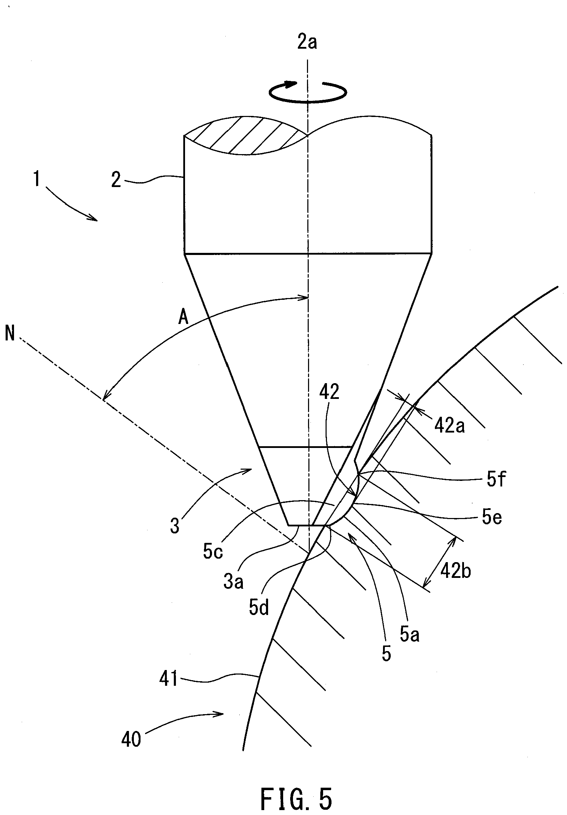

As shown in FIG. 5, the cutting tool 1 can form dimples 42 on the curved surface 41 of the workpiece 40. The curved surface 41 may have a convex shape, for example, a spherical partial shape or cylindrical partial shape. The workpiece 40 may be made of a steel material, such as carbon steel, rolled steel for general structures, chrome molybdenum steel, stainless steel, or cast iron. Alternatively, the workpiece 40 may be made of a resin material or non-ferrous metal, such as cobalt-chromium alloy, aluminum, an aluminum alloy, copper, or a copper alloy. The workpiece 40 may be a bone head of an artificial bone for an artificial joint that may be made of, for example, a ball and an inner ring of a bearing, a joint of a pipe, a cobalt-chromium alloy, or the like. The materials for the rod-shaped body 2 and the cutting edge portion 5 may be appropriately selected and set depending on the material of the workpiece 40.

As shown in FIG. 5, the axis 2a of the cutting tool 1 is set at a predetermined angle with respect to the workpiece 40. For example, the cutting tool 1 is set so that the axis 2a is inclined at an inclination angle A with respect to the normal line N of the curved surface 41 of the workpiece 40. The cutting tool 1 is set so that the radial tip end portion 5e has a predetermined depth 42a with respect to the curved surface 41. The cutting tool 1 rotates about the axis 2a. The cutting edge 5a cuts the curved surface 41 in a predetermined rotation angle region and moves away from the curved surface 41 in another rotation angle region. As a result, the cutting edge 5a intermittently cuts the curved surface 41. Each time the cutting tool 1 makes one rotation, one cutting edge 5a cuts the curved surface 41 once to form the dimple 42.

As shown in FIG. 5, the inclination angle A may be set to be smaller than 90°, for example, less than or equal to 75°, less than or equal to 60°, or less than or equal to 45° so that the cutting edge 5a moves away from the curved surface 41. The inclination angle A may be set to be greater than 0°, for example, greater than or equal to 1°, greater than or equal to 2°, or greater than or equal to 5° so that the cutting edge 5a cuts the curved surface 41 and so that the dimples 42 preferably have a length 42b in a predetermined cutting direction. The predetermined depth 42a may be set so that the maximum depth of the dimples 42 will be, for example, 0.001 mm to 0.1 mm.

As shown in FIG. 10, the dimples 42 are formed in an elliptical shape having a longitudinal direction in the cutting direction by the cutting edge 5a. Each of the dimples 42 is very small and may have a length 42b in the cutting direction, for example, of 0.5 mm to 1 mm. The cutting direction is the direction in which the cutting edge 5a (see FIG. 5) approaches the curved surface 41. For example, the cutting direction may be a direction defined by connecting a point where a predetermined portion of the cutting edge 5a reaches the curved surface 41 and a point where the predetermined portion of the cutting edge 5a is moved away from the curved surface 41. Each of the dimples 42 has a width orthogonal to the cutting direction. The maximum width 42c, which is the maximum of width, may be shorter than the length 42b, for example, of 0.01 mm to 0.5 mm.

The cutting edge 5a shown in FIG. 5 enters the workpiece 40 diagonally with respect to the curved surface 41, and enters gradually deeper into the workpiece 40. Then, the cutting edge 5a gradually moves out from the workpiece 40, cuts the workpiece 40 shallowly, and moves diagonally from the curved surface 41. Therefore, the dimples 42 are shallower at both ends and deeper at approximately the center in the length direction. For example, dimples formed by irradiating a machining surface with a laser, or dimples formed by shot peening have a sharp angle that is substantially perpendicular to the machining surface. Compared to such dimples, the dimples 42 do not have a sharp angle with respect to the curved surface 41. Therefore, the squeeze effect due to the pressure of the liquid filled in the dimple 42 is more easily obtained.

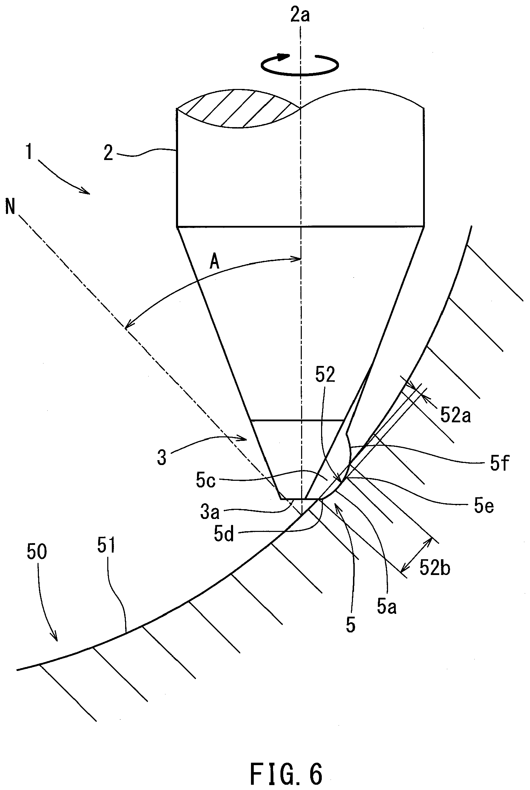

As shown in FIG. 6, the cutting tool 1 can also form dimples 52 on a workpiece 50 having a concave curved surface 51. The curved surface 51 has a concave, for example, a spherical partial shape or a partial shape of a circular inner periphery. The workpiece 50 may be made of a resin material, such as polyethylene. Alternatively, the workpiece 50 may be made of a metal material such as a steel material, aluminum, an aluminum alloy, copper, or a copper alloy. The workpiece 50 may be, for example, an outer ring of a bearing or a liner (insert) of an artificial bone made of polyethylene that engages with a bone head of an artificial joint. The cutting tool 1 is set so as to have an inclination angle A and so that the radial tip end portion 5e has a predetermined depth 52a with respect to the curved surface 51.

The workpiece 40 shown in FIG. 5 may be used as, for example, a bone head, and the workpiece 50 shown in FIG. 6 may be used as a liner that engages with, for example, the workpiece 40. The workpieces 40, 50 engage each other and are relatively rotatable. The friction generated when the workpieces 40, 50 are relatively rotated is reduced by the dimples 42, 52. For example, the dimples 42, 52 are filled with a liquid such as water, body fluid, or oil. When the workpieces 40, 50 slide against each other, the liquid is discharged out of the dimples 42, 52 at a high pressure and in between the workpieces 40, 50 due to the squeeze effect. This pressure may hinder the workpieces 40, 50 from coming into contact with each other, which reduces their frictional resistance. For example, when the workpiece 40 and the workpiece 50 slide, abrasion debris is generated from one or both of the workpieces 40, 50. This abrasion debris is held in the dimples 42, 52.

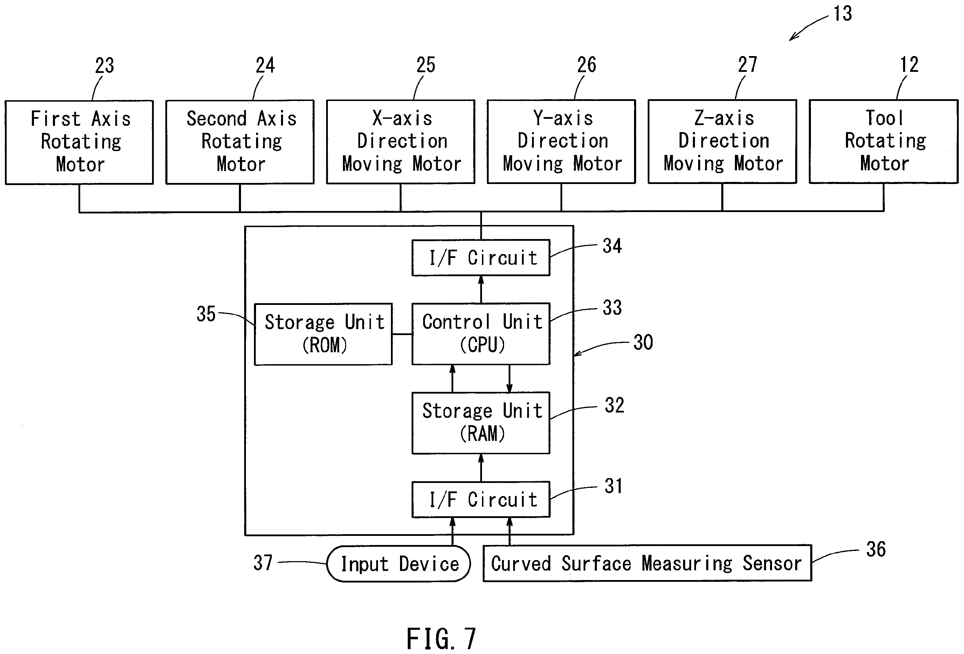

As shown in FIG. 1, the processing apparatus 10 includes a spindle 11 extending substantially in a Z-direction and a tool rotating motor 12 for rotating the spindle 11 around the Z-axis. An attachment portion on which the cutting tool 1 is attached is provided at the leading end of the spindle 11. The spindle 11 uses the tool rotating motor 12 to rotate the cutting tool 1 around the axis 2a. The motion or rotation of the spindle 11 is controlled by a control unit 33 accommodated in a controller 30, which is shown in FIG. 7.

As shown in FIG. 1, the workpiece holding device 20 includes a base 21 and a table 22 movably or turnably mounted on an upper side of the base 21. The workpiece 40 is held in the workpiece holding piece 22a provided on an upper side of the table 22. The table 22 moves or turns together with the workpiece 40 while the workpiece 40 is held in the workpiece holding piece 22a.

As shown in FIG. 1, the workpiece holding device 20 includes a first-axis rotating motor 23 and a second-axis rotating motor 24. The table 22 turns around the first axis 20a with respect to the base 21 using the first axis rotating motor 23. The first axis 20a passes through the spherical center 41a of the spherical partial shape of the curved surface 41 and the rotation center 22b of the table 22. The table 22 turns around the second axis 20b with respect to the base 21 using the second axis rotating motor 24. The second axis 20b extends in a Y-axis direction, passes through the rotation center 22b of the table 22, and orthogonally intersects the first axis 20a. The processing apparatus 10 or the spindle 11 uses, for example, a feed screw mechanism, a rack and pinion mechanism, X-axis, Y-axis, and Z-axis direction moving motors 25, 26, and 27 (see FIG. 7) so as to move in the X-axis direction, the Y-axis direction, and the Z-axis direction with respect to the base 21. The movement or turn of the processing apparatus 10 and the table 22 is controlled by the control unit 33 accommodated in the controller 30, shown in FIG. 7. The X-axis direction, the Y-axis direction, and the Z-axis direction are orthogonal to each other.

As shown in FIG. 7, the movement or rotation of each member of the processing apparatus 10 and the workpiece holding device 20 is controlled by the control unit (CPU) 33 in the controller (PC) 30 via an I/F circuit 34. A storage unit (ROM) 35 stores programs, algorithms, and data including commands necessary for executing the control unit 33. Data related to the processing mode, coordinate data of the workpiece 40, data related to the rotation speeds of each of the motors 12, 23 to 27, etc. are input via an input device 37 such as a keyboard. The input data is stored in the storage unit (RAM) 32 via the I/F circuit 31. The coordinate data of the workpiece 40 is corrected by measuring a plurality of predetermined points on the curved surface 41 in advance with a curved surface measuring sensor 36, such as a touch probe. The control unit 33 transmits a predetermined drive command to each of the motors 12, 23 to 27 based on the stored data, and each of the motors 12, 23 to 27 performs a predetermined drive operation based on transmitted signals.

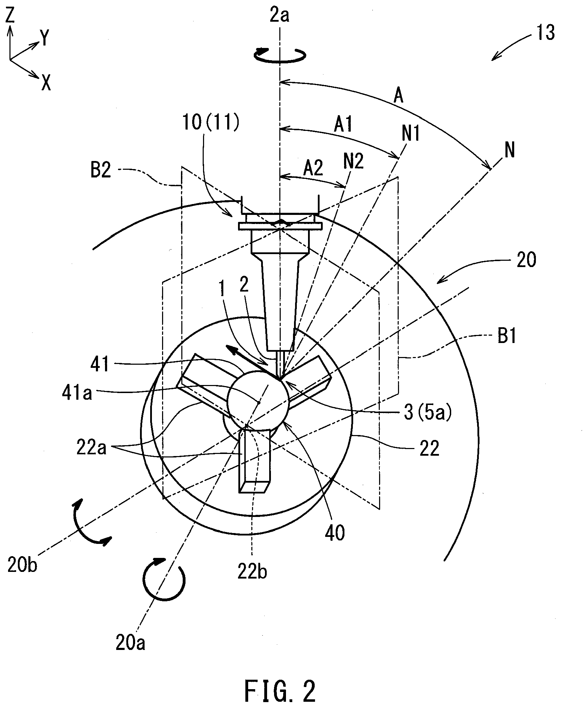

As shown in FIG. 2, the cutting tool 1 is tilted at a tilt angle A1 with respect to the normal line N1 of the curved surface 41 on the virtual plane B1 that is orthogonal to the cutting direction and passing through the axis 2a. The cutting tool 1 is tilted at a lead angle A2 with respect to the normal line N2 of the curved surface 41 on the virtual plane B2 which is parallel to the cutting direction and passes through the axis 2a.

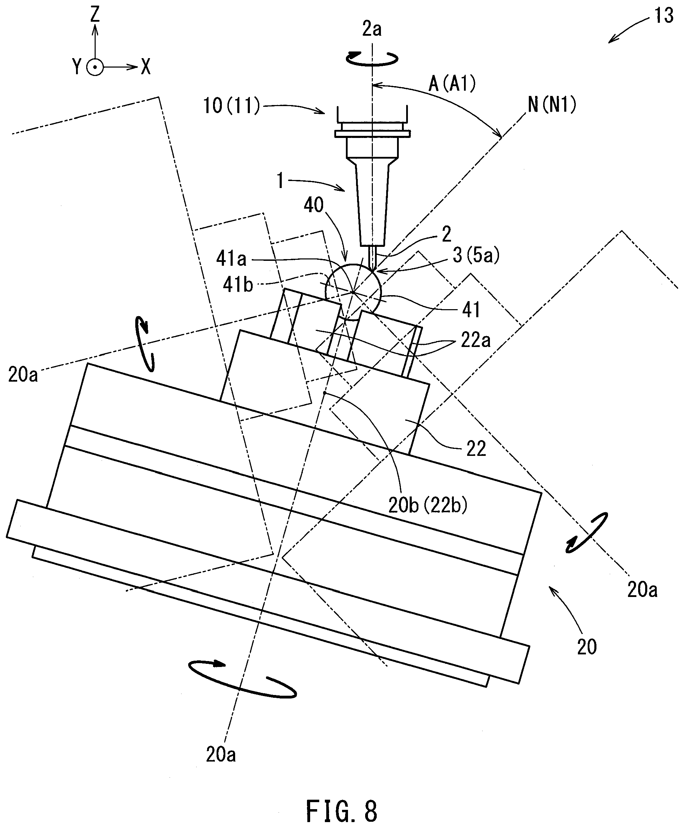

As shown in FIG. 8, one method of forming dimples is to move or turn the processing apparatus 10 and the table 22 so that the tilt angle A1 of the axis 2a becomes constant. The table 22 turns around the first axis 20a and the second axis 20b using the first axis rotating motor 23 and the second axis rotating motor 24 (see FIG. 1). The processing apparatus 10 moves along a plane orthogonal to the Y-axis and parallel to the virtual plane that passes through the first axis 20a utilizing the X-axis direction moving motor 25 and the Z-axis direction moving motor 27 (see FIG. 7).

As shown in FIG. 8, the tip end 3 is set such that it is located at a predetermined distance from the curved surface 41 and is adjacent to the first axis 20a. The tilt angle A1 of the axis 2a is set at a predetermined angle. The cutting tool 1 is rotated around the axis 2a at a predetermined speed, and the table 22 is turned around the center of the first axis 20a. The cutting tool 1 and the table 22 are relatively moved utilizing each of the motors 24, 25, 27 (FIG. 7) such that the tip end 3 gradually moves away from the center of the axis of the first axis 20a up to the outermost peripheral edge 41b of the curved surface 41 and gradually approaches when the tip end 3 has moved further than the outermost peripheral edge 41b while the distance between the tip end 3 and the curved surface 41 and the tilt angle A1 of the axis 2a are maintained constant. The outermost peripheral edge 41b is the peripheral edge farthest from the first axis 20a of the curved surface 41 and corresponds to a so-called spherical equator. As a result, the tip end 3 moves on the locus of the spiral C shown in FIG. 10 with respect to the curved surface 41.

The cutting edge 5a shown in FIG 5 repeats contacting with and moving away from the curved surface 41 each time the cutting tool 1 rotates about the center of the axis 2a. Since the cutting edge 5a cuts the curved surface 41 when it comes into contact with the curved surface 41, the curved surface 41 is cut intermittently. As shown in FIG. 10, an elliptical dimple 42 is formed on the curved surface 41 by one cutting of the cutting edge 5a. The cutting edge 5a (see FIG. 8) moves relative to the curved surface 41 along the spiral C. As a result, the plurality of dimples 42 are formed on the curved surface 41 along the spiral C. The point where the cutting edge 5a reaches the curved surface 41 and starts one cutting, and the point where the cutting edge 5a is moved away from the curved surface 41 and finishes one cutting are both aligned on the spiral C. As a result, each dimple 42 is formed along a direction that is the longitudinal direction is a spiral C extending direction.

The cutting edge 5a shown in FIG. 8 comes into contact with the curved surface 41 at the same location where the tilt angle A1 of the axial center 2a is constant, and comes into contact with the curved surface 41 at, for example, the radial tip end portion 5e (see FIG. 5). By repeatedly cutting the curved surface 41 at the same location of the cutting edge 5a, a plurality of dimples 42 having the same elliptical shape and the same size can be formed on the curved surface 41.

The relative moving speed and moving amount of the cutting tool 1 shown in FIG. 8 with respect to the curved surface 41 may be appropriately changed or set to be constant. For example, the turning speed of the table 22 around the first axis 20a may be set in accordance with the distance between the tip end 3 and the first axis 20a. When the tip end 3 is adjacent to the first axis 20a, the table 22 is turned at high speed around the first axis 20a. As the tip end 3 moves away from the first axis 20a, the turning speed of the table 22 around the first axis 20a is reduced. This allows a plurality of dimples to be arranged at substantially equal intervals such that the dimples 42 are evenly arranged on the curved surface 41. Alternatively, the turning speed of the table 22 around the first axis 20a is set to be constant. This allows the plurality of dimples 42 to be arranged at small intervals on the curved surface 41 around the first axis 20a and at large intervals on the curved surface 41 distanced from the first axis 20a.

The depth 42a (see FIG. 5) of the plurality of dimples 42 can be changed by changing the cutting depth as the cutting tool 1 shown in FIG. 8 moves relative to the curved surface 41. For example, machining is started from the first axis 20a, the cutting depth is increased in the vicinity thereof, and the cutting depth is gradually reduced as the cutting tool 1 relatively moves. As a result, the size of the plurality of dimples 42 is large on the curved surface 41 around the first axis 20a and small on the curved surface 41 distanced from the first axis 20a.

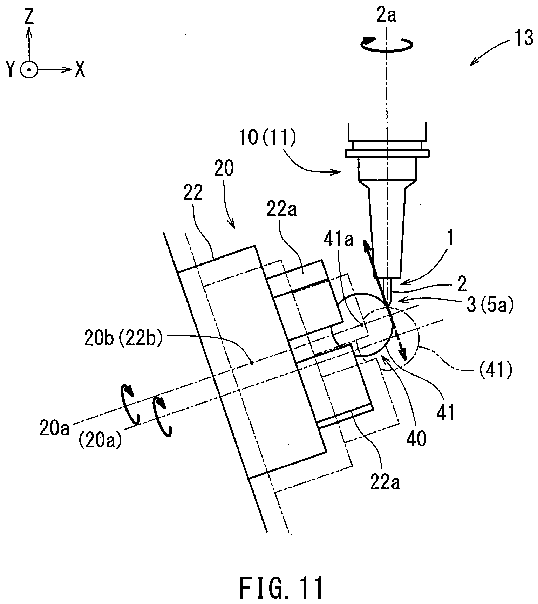

As shown in FIG. 11, another method of forming dimples is to move or turn/rotate the table 22, the processing apparatus 10, and/or the spindle 11 so that the lead angle A2 (see FIG. 2) of the axis 2a is constant. The table 22 turns around the first axis 20a using the first axis rotating motor 23 (see FIG. 1). The table 22 is tilted at a predetermined angle about the second axis 20b using the second axis rotating motor 24 (see FIG. 1). Further, the processing apparatus 10 or the spindle 11 moves along a plane orthogonal to the Y axis and parallel to the virtual plane passing through the first axis 20a via each of the motors 25 to 27 (see FIG. 7).

As shown in FIG. 11, the tip end 3 is set such that it is located at a predetermined distance from the curved surface 41 and is adjacent to the first axis 20a. The lead angle A2 (see FIG. 2) of the axis 2a is set to a predetermined angle. In FIG. 11, the normal line N2 (see FIG. 2) overlaps the axis 2a in the direction orthogonal to the sheet surface, so that the lead angle A2 is not visible in FIG. 11. The cutting tool 1 is rotated around the axis 2a at a predetermined speed, and the table 22 is turned around the first axis 20a. The tip end 3 is gradually moved away from the center of the first axis 20a via each of the motors 24 to 27 (see FIG. 7) so as to move relative to the curved surface 41 along the spiral C (see FIG. 10) while maintaining the distance between the tip end 3 and the curved surface 41, and the lead angle A2 of the axis 2a constant.

The cutting edge 5a shown in FIG. 5 cuts the curved surface 41 once each time the cutting tool 1 rotates about the axis 2a to form the dimples 42. The plurality of dimples 42 are formed along the spiral C shown in FIG. 10. The point at which the cutting edge 5a reaches the curved surface 41 and starts one cutting is located on an inner peripheral side (or outer peripheral side) of the spiral C. The point at which the cutting edge 5a moves away from the curved surface 41 and finishes one cutting is located on the outer peripheral side (or inner peripheral side) of the spiral C. As a result, each dimple 42 is inclined in the longitudinal direction with respect to the extending direction of the spiral C as shown by the broken line in FIG. 10. The cutting edge 5a comes into contact with the curved surface 41 at the same location since the lead angle A2 (see FIG. 2) of the axis 2a is constant. Therefore, the cutting edge 5a may form a plurality of dimples 42 having the same elliptical shape and the same size on the curved surface 41, as was in the case where the tilt angle A1 (see FIG. 2) of the axis 2a was constant.

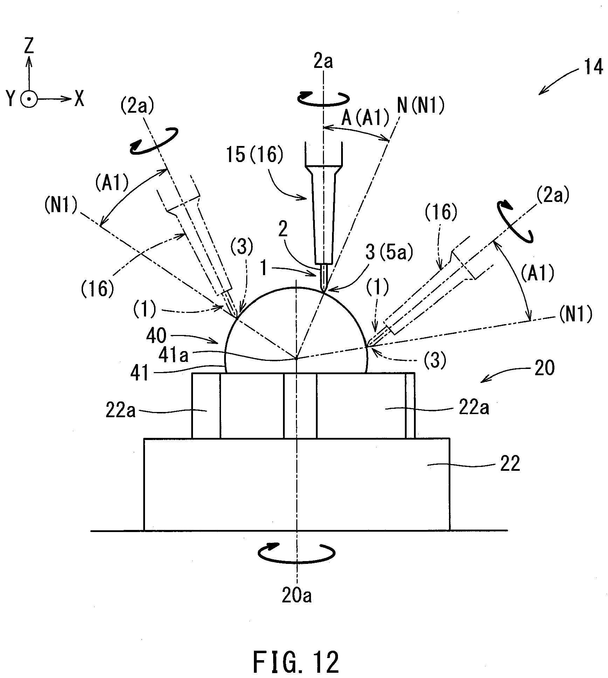

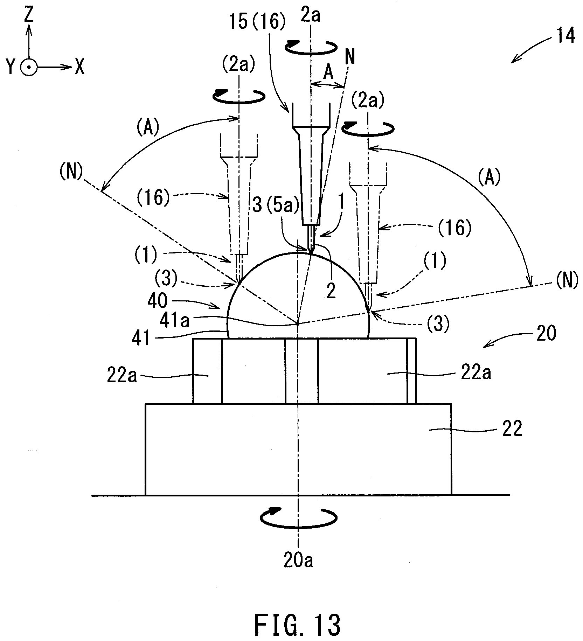

Next, another exemplary embodiment of the present disclosure will be described with reference to FIGS. 12 and 13. In this embodiment, the dimple processing machine 14 shown in FIGS. 12 and 13 is used instead of the dimple processing machine 13 shown in FIG. 1. The dimple processing machine 14 has a processing apparatus 15 instead of the processing apparatus 10. The processing apparatus 15 has a spindle 16 that rotates about the axis. As shown in FIG. 12, in one method of forming dimples, the cutting tool 1 is moved and the axis 2a is inclined. The processing apparatus 15 or the spindle 16 can move in the X-axis direction, the Y-axis direction, and the Z-axis direction with respect to the workpiece holding device 20. In addition, the inclination direction of the processing apparatus 15 or the spindle 16 can be changed. An attachment portion on which the cutting tool 1 is attached is provided at the leading end of the spindle 16. The spindle 16 rotates the cutting tool 1 around the axis 2a. The rotation or movement or inclination of the processing apparatus 15 or the spindle 16 is controlled by the control unit 33, shown in FIG. 7.

As shown in FIG. 12, the table 22 turns around the first axis 20a using the first axis rotating motor 23 (see FIG. 1). The spindle 16 moves along a plane orthogonal to the Y axis and parallel to the virtual plane passing through the first axis 20a so that the tip end 3 is located a predetermined distance from the curved surface 41. Further, the spindle 16 is tilted so that the tilt angle A1 of the axis 2a has a predetermined angle. The cutting tool 1 rotates about the axis 2a. The cutting edge 5a cuts the curved surface 41 once each time the cutting tool 1 makes one rotation to form dimples 42 (see FIG. 10). The cutting edge 5a comes into contact with the curved surface 41 at the same relative location by maintaining the tilt angle A1 of the axial center 2a constant.

As shown in FIG. 13, in another method of forming dimples, the cutting tool 1 is moved using the dimple processing machine 14 with the axis 2a extending direction extending in the same as the Z-axis direction. The table 22 turns around the first axis 20a using the first axis rotating motor 23 (see FIG. 1). The spindle 16 moves along a plane orthogonal to the Y axis and parallel to the virtual plane passing through the first axis 20a such that the tip end 3 is located a predetermined distance from the curved surface 41. The cutting tool 1 rotates about the axis 2a extending in the Z-axis direction. The cutting edge 5a cuts the curved surface 41 once each time the cutting tool 1 rotates to form the dimples 42 (see FIG. 10).

As shown in FIG. 13, the inclination angle A of the axis 2a with respect to the curved surface 41 increases or decreases as the cutting tool 1 is moved by the spindle 16. As shown in FIG. 3, the cutting edge 5a has a predetermined length, in the longitudinal direction, from a cutting edge end portion 5d to the cutting edge base portion 5f. As shown in FIG. 5, the portion of the cutting edge 5a that comes into contact with the curved surface 41 changes in the longitudinal direction as the inclination angle A increases or decreases. For example, when the inclination angle A is small, the cutting edge 5a comes into contact with the curved surface 41 at a portion close to the cutting edge end portion 5d. When the inclination angle A is large, it comes into contact with the curved surface 41 at a portion close to the radial tip end portion 5e or the cutting edge base portion 5f. The portion where the cutting edge 5a comes into contact with the curved surface is shifted from the tip end side to the base portion side of the tip end 3 as the inclination angle A increases.

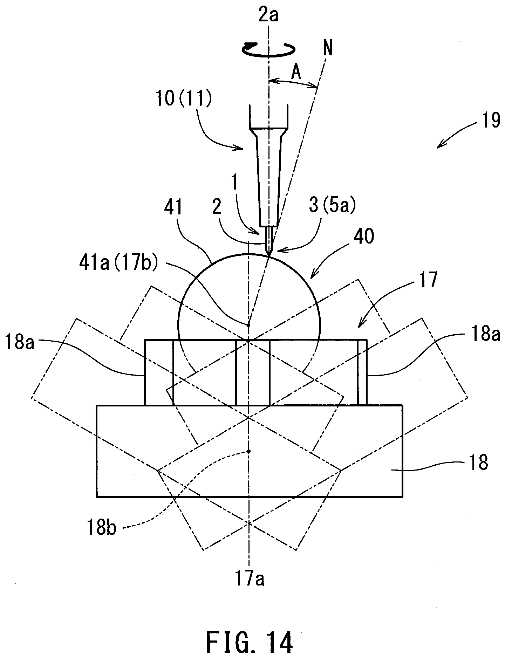

Next, another exemplary embodiment of the present disclosure will be described with reference to FIG. 14. In this embodiment, the dimple processing machine 19 shown in FIG. 14 is used instead of the dimple processing machine 13 shown in FIG. 1. The dimple processing machine 19 includes a workpiece holding device 17 instead of the workpiece holding device 20. The workpiece holding device 17 includes a base (not shown) and a table 18 turnably mounted on the upper side of the base. The table 18 includes a workpiece holding piece 18a on the upper surface for holding the workpiece 40. In one method of forming dimples, the workpiece 40 is rotated about two axes and the cutting tool 1 is further rotated around another axis 2a. The table 18 turns around a first axis 17a that passes through a spherical center 41a and the rotation center 18b of the table 18. The table 18 turns around a second axis 17b extending in the Y-axis direction through the rotation center 18b. The first axis 17a and the second axis 17b are orthogonal to each other. The rotation of the table 18 and the cutting tool 1 are controlled by the control unit 33, shown in FIG 7.

As shown in FIG. 14, the tip end 3 is set so as to be located at a predetermined distance from the curved surface 41. The inclination angle A of the axis 2a is set to a predetermined angle. The cutting tool 1 is rotated around the axis 2a at a predetermined speed, and the table 18 is turned around the first axis 17a at a high speed. Further, the table 18 is turned around the second axis 17b at a relatively low speed. As a result, a plurality of dimples 42 (see FIG. 10) are formed on the spherical curved surface 41 centered on the spherical center 41a.

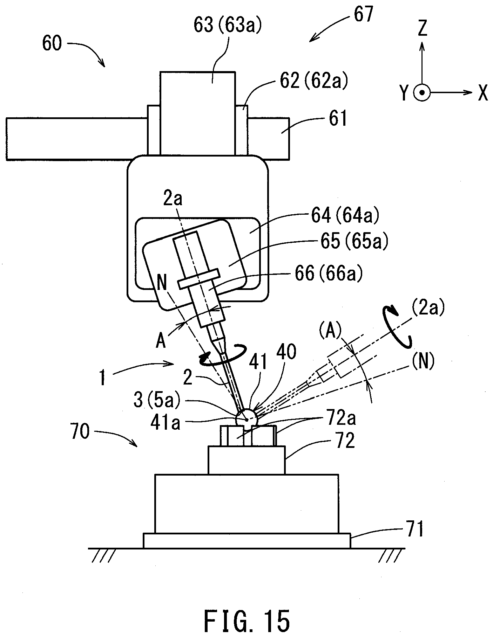

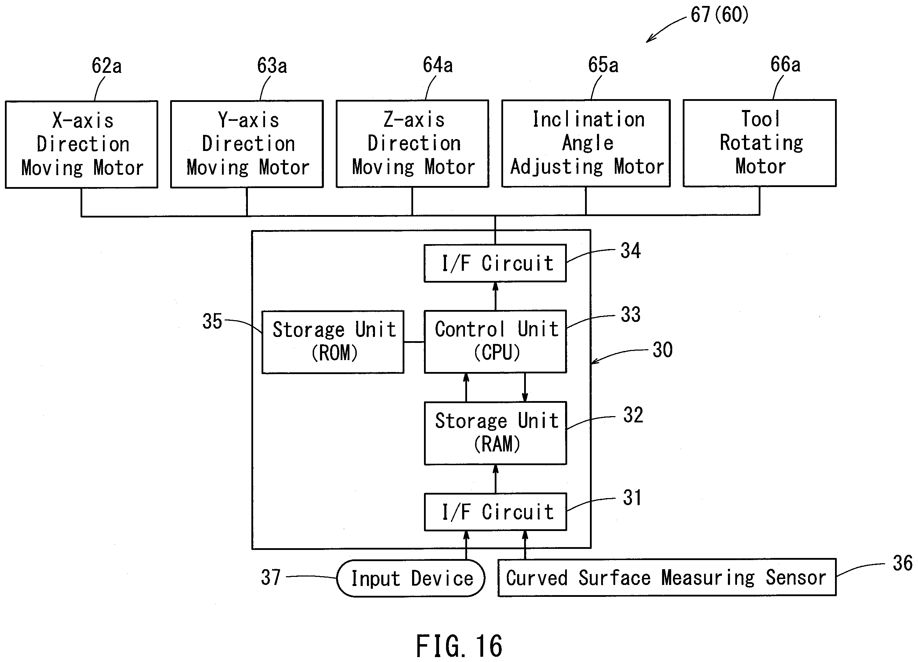

Next, another exemplary embodiment of the present disclosure will be described with reference to FIGS. 15 and 16. In this embodiment, a dimple processing machine 67 is used instead of the dimple processing machine 13 shown in FIG 1. The dimple processing machine 67 includes a processing apparatus 60 and a workpiece holding device 70 instead of the processing apparatus 10 and the workpiece holding device 20. In one method of forming dimples, the workpiece 40 is not rotated while the cutting tool 1 is moved or the axis 2a is inclined.

As shown in FIG. 15, the processing apparatus 60 includes an X-axis guide 61, an X-direction moving member 62 capable of moving along the X-axis guide 61, a Y-direction moving member 63 that moves in the Y-axis direction with respect to the X-direction moving member 62, and a Z-direction moving member 64 that moves in the Z-axis direction with respect to the Y-direction moving member 63. The X-axis guide 61 is held on a support base (not shown) and extends in the X-axis direction. The X-direction moving member 62 moves with respect to the X-axis guide 61 using, for example, a feed screw mechanism, a rack and pinion mechanism, and an X-axis direction moving motor 62a. The Y-direction moving member 63 and the Z-axis moving member 64 move in the Y-axis direction and the Z-axis direction with respect to the X-direction moving member 62 using, for example, a feed screw mechanism, a rack and pinion mechanism, a Y-axis direction moving motor 63a, and a Z-axis direction moving motor 64a.

As shown in FIG. 15, the processing apparatus 60 may include a swing member 65 mounted to the Z-direction moving member 64 in an angle adjustable manner, and a spindle 66 provided on the swing member 65 in an axially rotatable manner. The swing member 65 swings in the X-axis direction or Y-axis direction with respect to the Z-direction moving member 64 using an inclination angle adjusting motor 65a. An attachment portion, to which the cutting tool 1 is attached, is provided at the leading end of the spindle 66. The spindle 66 serves to rotate the cutting tool 1 about the axis 2a using the tool rotating motor 66a. As shown in FIG. 16, each of the motors 62a to 66a for the processing apparatus 60 is controlled by the control unit 33 that is accommodated in the controller 30. As shown in FIG. 15, the workpiece 40 is held on the workpiece holding device 70. The workpiece holding device 70 includes a base 71 and a table 72 mounted on the upper side of the base 71. The workpiece 40 is held on the workpiece holding piece 72a that is provided on the upper side of the table 72.

As shown in FIG. 15, the spindle 66 moves in the X-axis, Y-axis, and Z-axis directions such that the tip end 3 is located a predetermined distance from the curved surface 41. Further, the spindle 66 is inclined such that the axis 2a is inclined at a predetermined inclination angle A. The cutting tool 1 rotates about the axis 2a. The cutting edge 5a cuts the curved surface 41 once each time the cutting tool 1 rotates to form the dimples 42 (see FIG. 10). Without rotating the workpiece, the cutting tool or workpiece is moved in the three directions orthogonal to each other so that the tip end of the cutting tool follows the curved surface of the workpiece. The cutting edge forms dimples on the curved surface each time the cutting tool rotates.

As described-above, in the dimple processing method for forming dimples 42 on the curved surface 41 of the workpiece 40, the cutting tool 1 having a cutting edge 5a on the rod-shaped body 2, as shown in FIGS. 8 and 11, is rotated about the axis 2a. The workpiece 40 is rotated about the first axis 20a. The cutting tool 1 or the workpiece 40 moves on a virtual plane orthogonal to the Y axis and through which the first axis 20a passes or on a plane parallel to the virtual plane such that the tip end 30 of the cutting tool 1 follows the curved surface 41 of the workpiece 41. The cutting edge 5a forms dimples 42 (see FIG. 10) on the curved surface 41 each time the cutting tool 1 rotates.

Therefore, the cutting tool 1 or the workpiece 40 moves in two dimensions on the plane. The cutting tool 1 is rotated about the axis 2a and the workpiece 40 is rotated about the first axis 20a. This causes a plurality of dimples 42 to be formed on the curved surface 41 in a short period of time. In addition, the depth 42a of the dimples 42 (see FIG. 5) can be made to be substantially constant and the approach angle can be reduced. A substantially constant depth 42a facilitates the reproduction of a plurality of dimples 42 having the same shape and the same size. A reduced approach angle reduces burrs, heat, and residual stress that may be generated during processing.

As shown in FIG. 15, the cutting tool 1 having the cutting edge 5a on the rod-shaped body 2 is rotated around the axis 2a of the rod-shaped body 2. Without rotating the workpiece 40, the cutting tool 1 or workpiece 40 is moved in the X-axis, Y-axis, and the Z-axis directions that are orthogonal to each other such that the tip 3 end of the cutting tool 1 follows the curved surface 41 of the workpiece 40. The cutting edge 5a forms dimples 42 on the curved surface 41 each time the cutting tool 1 rotates (see FIG. 10).

Therefore, the cutting tool 1 or the workpiece 40 is moved in three dimensions. Only the cutting tool 1 is rotated around the axis 2a. And the workpiece 40 is not rotated. As a result, a plurality of dimples 42 can be formed on the curved surface 41 in a short period of time. In addition, the depth 42a of the dimple 42 (see FIG. 5) can be made substantially constant and the approach angle can be reduced.

As shown in FIG. 5, the cutting edge 5a continuously extends in an arc shape along the longitudinal direction of the rod-shaped body 2. As shown in FIG. 13, the rod-shaped main body 2 is moved relative to the workpiece 40 while maintaining the angle of the rod-shaped body 2 of the cutting tool 1 with respect to the workpiece 40. As a result, the contact point of the cutting edge 5a on the curved surface 41 changes. Therefore, it is possible to avoid concentrating the force on a part of the cutting edge 5a by changing the contact point. As a result, the life of the cutting edge 5a can be extended.

As shown in FIGS. 8, 11, and 12, the cutting tool 1 is inclined with respect to the workpiece 40 such that the tilt angle A1 or the lead angle A2 (see FIG. 2) of the rod-shaped body 2 with respect to the plane where the cutting tool 1 comes in contact with the curved surface 41 of the workpiece 40 is configured to be constant. This causes the contact point of the cutting edge 5a on the curved surface 41 to be constant. Therefore, the approach angle or the cutting depth of the cutting edge 5a with respect to the curved surface 41 can be made constant. As a result, a plurality of dimples 42 (see FIG. 10) having the same shape and the same size can be repeatedly formed.

As shown in FIG. 14, the curved surface 41 has a spherical partial shape, and the workpiece 40 is rotated about the first axis 17a that passes through the spherical center 41a of the spherical shape. The workpiece is rotated about the second axis 17b that is orthogonal to the first axis 17a. The cutting tool 1 having a cutting edge 5a on the rod-shaped body 2 is rotated about the axis 2a. The tip end 3 of the cutting tool 1 moves along a part of the spherical shape of the curved surface 41 of the workpiece 40. The cutting edge 5a forms dimples 42 (see FIG 10) on the curved surface 42 each time the cutting tool 1 rotates.

Therefore, the workpiece 40 is rotated around the two axes, while the cutting tool 1 is rotated around the axis 2a, such that they are rotated around a total of three axes. As a result, a plurality of dimples 42 can be formed on the spherical portion of the curved surface 41 in a short period of time. In addition, the depth of the dimples 42 can be made substantially constant and the approach angle can be reduced.

The cutting depth is changed in accordance with the relative moving speed and amount of movement of the cutting tool 1 shown in FIG. 8 with respect to the curved surface 41, or as the cutting tool relatively moves on the curved surface. As a result, the interval and depth 42a (see FIG. 5) of the plurality of dimples 42 (see FIG. 10) can be set to a predetermined size. Therefore, the interval and depth 42a of the plurality of dimples 42 can be changed for each portion of the curved surface 41.

Various modifications may be made to each of the above-described exemplary embodiments. The processing method of each dimple may also be applied when the dimples 52 are formed on a concave curved surface 51 of the workpiece 50 shown in FIG. 6. The processing method of each dimple may also be applied when the dimples are formed on the curved surface having both a convex partial shape and a concave partial shape. For example, nearly perfect circular dimples or elliptical dimples having a longitudinal direction in a direction orthogonal to the cutting direction may be formed on the curved surface 41 shown in FIG. 10 by increasing the rotation speed of the cutting tool 1 shown in FIG. 1 about the axis 2a etc. For example, successive dimples with some parts of the plurality of elliptical shape overlapped along the cutting direction may be formed by reducing the rotation speed of the workpiece 40 shown in FIG. 1 about the first axis 20a, etc. For example, the dimples 42 may be arranged on the curved surface 41 at unequal intervals by changing the rotation speed of the workpiece 40 about the first axis 20a, etc.

The curved surface 41 may have, for example, a hemispherical shape, or a part having a spherical shape, or may also have a variety of other shapes. According to the processing method in FIGS. 8 and 9, the cutting tool 1 moves on the virtual plane being an apex to the bottom surface of the hemispherical shape. This enables the dimples 42 to be formed near the apex. Alternatively, the cutting tool 1 may be moved on the virtual plane that does not pass through the apex. In this case, the dimples 42 cannot be formed near the apex but the dimples 42 can be formed in the other areas of the curved surface 41.

The relative movement track of the tip end 3 on the curved surface 41 is not limited to the spiral C illustrated in FIG. 10, and may be appropriately changed. For example, the workpiece 40 shown in FIG. 12 may be rotated once around the first axis 20a extending in the Z-axis direction, and the workpiece 40 may temporarily stop being rotating. The cutting tool 1 is moved relative to the curved surface 41 along a virtual plane orthogonal to the Y axis, and the workpiece 40 is rotated once again about the first axis 20a. By repeating this movement, the tip end 3 relatively moves on the curved surface 41 in a plurality of concentric circles manner centered on the first axis 20a. For example, the cutting tool 1 may be moved relative to the curved surface 41 such that the dimples 42 start to be formed while the tip end 3 is positioned at the point farthest away from the first axis 20a of the curved surface 41, and are finished at the point adjacent to the first axis 20a of the curved surface 41.

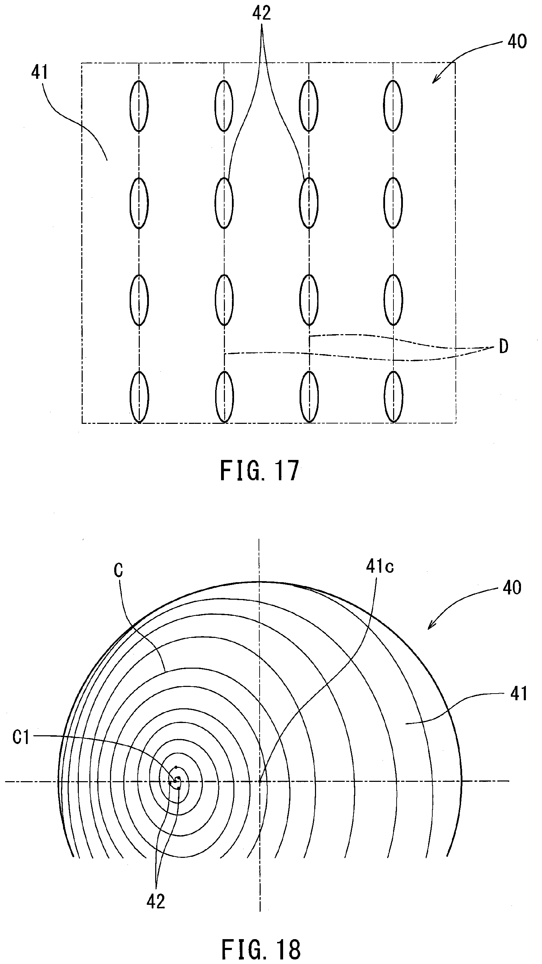

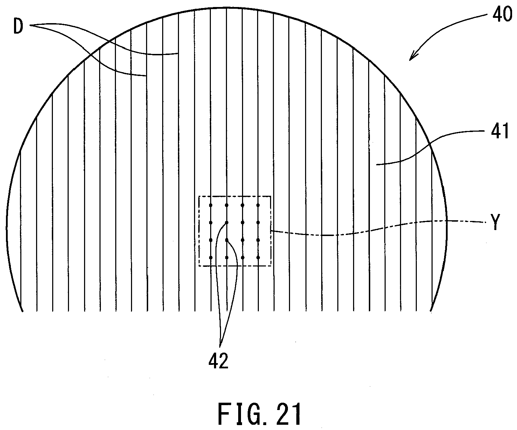

For example, the tip end 3 may be positioned at the point farthest away from the first axis 20a of the curved surface 41 in an X-axis direction or Y-axis direction without turning the table 22. The tip end 3 is relatively moved from that position to the point that is orthogonal to the X-axis or the Y-axis and line-symmetrical with the first axis 20a. The cutting tool 1 is moved in the X-axis direction or the Y-axis direction, and the tip portion 3 is relatively moved again to the point that is orthogonal to the X-axis or the Y-axis and is line-symmetrical with the first axis 20a. As shown in FIGS. 17 and 21, the dimples 42 can be arranged on the curved surface 41 in a lattice pattern aligned along a plurality of lines D arranged parallel at substantially equal intervals in the X-axis direction or the Y-axis direction by repeating this movement. For example, the apex 42c of the spherical shape of the curved surface 41 (see FIG. 18) may not be arranged on the first axis 20a (FIG. 8), and the workpiece 40 may be set being inclined at a predetermined angle. As shown in FIG. 18, the center C1 of the spiral C and the apex 41c of the curved surface 41 may thereby be displaced from each other.

As shown in FIGS. 8 and 11, the dimples may be formed in an intermediate method between a cutting method where the tilt angle A1 or the lead angle A2 of the axis 2a (see FIG. 2) is constant and a cutting method where the inclination angle A of the axis 2a changes as shown in FIG. 13. For example, the table 22 shown in FIG. 1 may be turned around the second axis 20b within a predetermined minute angle range. This causes the portion where the cutting edge 5a comes in contact with the curved surface 41 to become slightly displace. The tool life can be extended by avoiding using the same portion the cutting edge 5a. Further, since the displacement of the portion where the cutting edge 5a comes in contact with the curved surface 41 is slight, any variation in the shape of the dimples to be formed can be reduced.

For example, the cutting edge portion 5 may have a triangular cutting edge projecting radially from the tip end 3. With this cutting edge, so-called spindle-shaped dimples may be formed on the curved surface 41. Each of the spindle-shaped dimple has a peripheral edge defined by connecting two points to form an arc shape on the both sides of a straight line that connects two points. For example, the cutting tool 1 may have a cutting edge that is positioned at a location offset from the axis 2a of the rod-shaped body 2 and projects in a direction of the axis 2a. With this cutting tool 1, so-called crescent-shaped dimples may be formed on the curved surface 41. Each of the crescent-shaped dimple has a peripheral edge defined by connecting two points to form an arc shape or a straight line on one side of the straight line that connects two points or on the straight line that connects the two points.

For example, the cutting tool 1 may have a plurality of cutting edges 5a on the tip end 3, as shown in FIG. 19. The plurality of cutting edges 5a may be arranged in a circumferential direction or in an axial direction. Six cutting edges 5a are aligned at equal intervals in a circumferential direction at the tip end 3 of FIG. 19. For example, the cutting edge portions 5, which may be positioned at substantially equal intervals, may have a polygonal cutting edge having three or more edges, or may have a cutting edge having a free-curved shape or a shape obtained by combining a plurality of shapes. A cutting edge having various types of shapes may be provided at the cutting tool 1, such that dimples having various types of shapes may be formed on the curved surface 41. Alternatively, a plurality of cutting tool having different shapes may be prepared. The rod-shaped body 2 may have a round rod shape or a rod shape with a polygonal cross-section. The rotating direction of the cutting tool 1 and a relatively moving direction of the curved surface 41 with respect to the cutting tool 1 may be appropriately selected. For example, the turning direction of the table 22 centered on the first axis 20a may be either one of the left or right directions. In other words, the processing of the dimples 42 may be either an upcut or a downcut.

## Claims

1. A dimple processing method for forming dimples on a curved surface of a workpiece comprising:
rotating a cutting tool having a cutting edge on a rod-shaped body around an axis of the rod-shaped body;
rotating the workpiece around a first axis; and
moving the cutting tool or the workpiece on a virtual plane through which the first axis passes or on a plane parallel to the virtual plane such that a tip end of the cutting tool follows the curved surface of the workpiece to allow the cutting edge to form a dimple on the curved surface each time the cutting tool rotates.

2. A dimple processing method for forming dimples on a curved surface of a workpiece comprising:
rotating a cutting tool having a cutting edge on a rod-shaped body around an axis of the rod-shaped body;
not rotating the workpiece; and
moving the cutting tool or the workpiece in three directions orthogonal to each other such that a tip end of the cutting tool follows the curved surface of the workpiece to allow the cutting edge to form a dimple on the curved surface each time the cutting tool rotates.

3. The dimple processing method as defined in claim 1 or claim 2, wherein:
the cutting edge continuously extends along a longitudinal direction of the rod-shaped body; and
the rod-shaped body is relatively moved with respect to the workpiece while maintaining an angle of the rod-shaped body of the cutting tool with respect to the workpiece to cause a contact point of the cutting edge to change with respect to the curved surface.

4. The dimple processing method as defined in claim 1 or claim 2, wherein the cutting tool is inclined with respect to the workpiece so as to maintain a constant angle of the rod-shaped body with respect to a plane where the cutting tool comes in contact with the curved surface of the workpiece such that a contact point of the cutting edge with the curved surface is made to be the same.

5. A dimple processing method for forming dimples on a curved surface of a workpiece comprising:
rotating the workpiece around a first axis passing through a spherical center of the workpiece having a curved surface, the curved surface having a spherical partial shape;
rotating the workpiece around a second axis orthogonal to the first axis; and
rotating a cutting tool having a cutting edge on a rod-shaped body around an axis of the rod-shaped body such that a tip end of the cutting tool moves along a part of the spherical shape of the workpiece to allow the cutting edge to form dimples on the curved surface each time the cutting tool rotates.

6. The dimple processing method as defined in any one of claims 1 to 5, wherein:
an interval between and a depth of a plurality of dimples are made to have a predetermined size by changing a moving speed and an amount of movement of the cutting tool relative to the curved surface, or by changing a cutting depth as the cutting tool relatively moves with respect to the curved surface.
